# EUROPÄISCHE PATENTSCHRIFT

(11) **EP 3 489 218 B1**
(45) Veröffentlichungstag und Bekanntmachung des Hinweises auf die Patenterteilung: **30.06.2021**
(21) Anmeldenummer: 18202901.7
(22) Anmeldetag: 26.10.2018
(51) Int. Cl.: C07C 221/00, C09B 1/514

(54) **VERFAHREN ZUR HERSTELLUNG VON PHENYLAMINOHYDROXYANTHRACHINONEN**
METHOD FOR THE PRODUCTION OF PHENYLAMINOHYDROXYANTHRACHINONES
PROCÉDÉ DE PRÉPARATION DE PHÉNYL AMINO HYDROXYANTHRAQUINONES

(30) Priorität: 24.11.2017 EP 17203513
(43) Veröffentlichungstag der Anmeldung: 29.05.2019
(73) Patentinhaber: LANXESS Deutschland GmbH, 50569 Köln (DE)
(72) Erfinder: BORST, Hans-Ulrich, 50189 Elsdorf (DE); SCHMAUSER, Sabine, 51371 Leverkusen (DE); MASSONG, Alexa, 51109 Köln (DE)

(56) Entgegenhaltungen:
- EP-A1- 1 074 586
- EP-A2- 0 284 580
- GB-A- 1 367 933
- JP-A- H09 291 238
- CHAO Y C ET AL: "BIS(ARYLAMINO)-DIHYDROXYANTHRAQUINONES: BLUISH GREEN DYES FOR SYNTHETIC POLYMER FIBRES", DYES AND PIGMENTS, ELSEVIER APPLIED SCIENCE PUBLISHERS. BARKING, GB, Bd. 19, Nr. 4, 1. Januar 1992 (1992-01-01) , Seiten 249-257, XP000324200, ISSN: 0143-7208, DOI: 10.1016/0143-7208(92)80030-Q
- FERGAL P. BYRNE ET AL: "Tools and techniques for solvent selection: green solvent selection guides", SUSTAINABLE CHEMICAL PROCESSES, Bd. 4, Nr. 1, 23. Mai 2016 (2016-05-23), XP055464727, DOI: 10.1186/s40508-016-0051-z
- Anonymous: "COMMENTS ON AN ANNEX XV DOSSIER FOR IDENTIFCATION OF A SUBSTANCE AS SVHC AND RESPONSES TO THESE COMMENTS 1-methyl-2-pyrrolidinone", , 29. November 2011 (2011-11-29), Seiten 1-53, XP055464725, Gefunden im Internet: URL:https://echa.europa.eu/documents/10162 /13638/rcom_methylpyrrolidone_20110510_en. rtf/af0a771d-ae52-4e0b-9e3a-4a3ba53aab00 [gefunden am 2018-04-05]

## Beschreibung

Die vorliegende Erfindung betrifft ein neues Verfahren zur Herstellung von 1,4-Diphenylamino-5,8-dihydroxyanthrachinonen durch Umsetzung von 1,4-Dichlor-5,8-dihydroxyanthrachinon mit Phenylaminen in Gegenwart spezieller Lösungsmittel.

Phenylaminohydroxyanthrachinone stellen eine wertvolle Klasse von Farbstoffen dar, die insbesondere für die Einfärbung von thermoplastischen Kunststoffen verwendet werden und sich dabei durch hohe Licht- und Wetterechtheiten auszeichnen.

Bekanntester Vertreter dieser Farbstoffklasse ist Solvent Green 28 (1,4-Bis(4-tert.-butylphenylamino)-5,8-dihydroxyanthrachinon (CAS-Nr. 4851-50-7, EINECS-Nr. 225-443-9).

Ein Verfahren zur Herstellung von Phenylaminohydroxyanthrachinonen durch Kondensation von Phenylaminen mit Chlorhydroxyanthrachinonen ist aus der EP-A 1 074 586 bekannt. Die Umsetzung der Chlorhydroxyanthrachinone mit den entsprechenden Aminen wird dort in Gegenwart spezieller Basen aus der Reihe der Alkaliphosphate und unter Verwendung spezieller, inerter Lösungsmittel wie N-Methylpyrrolidon, Chlorbenzol, 1,2-Dichlorbenzol, 1,2,4-Trichlorbenzol, Xylol, Nitrobenzol und Phenol durchgeführt. Als besonders bevorzugte Lösungsmittel werden N-Methylpyrrolidon und 1,2-Dichlorbenzol beschrieben.

Dieses Verfahren liefert die Phenylaminohydroxyanthrachinone in relativ guten Ausbeuten und die Restgehalte an Amin im Farbstoff liegt bei niedrigen Werten von unterhalb 600 ppm.

Allerdings entsprechen die koloristischen Eigenschaften solchermaßen hergestellter Produkte nicht mehr den aktuellen anwendungstechnischen Anforderungen.

Es besteht daher ein dringender Bedarf nach Herstellverfahren, die Phenylaminohydroxyanthrachinone mit verbesserten koloristischen Eigenschaften liefern. Aufgabe der vorliegenden Erfindung war es also ein verbessertes Verfahren zur Herstellung von 1,4-Diphenylamino-5,8-dihydroxyanthrachinonen bereit zu stellen.

Es wurde ein neues Herstellverfahren gefunden, welches 1,4-Diphenylamino-5,8-dihydroxyanthrachinone in sehr guten Ausbeuten, hoher Reinheit und mit verbesserten koloristischen Eigenschaften liefert.

Gegenstand der vorliegenden Erfindung ist ein Verfahren zur Herstellung von Verbindungen der Formel (I) 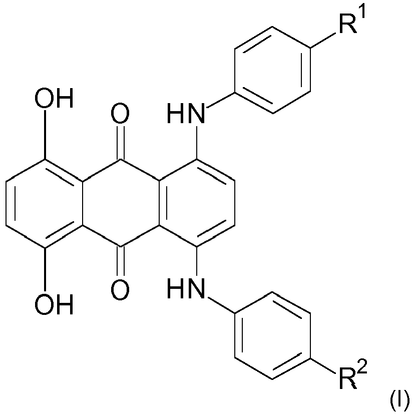 worin
R¹ und R² gleich sind und für C₁-C₆-Alkyl stehen,
durch Umsetzung von 1,4-Dichlor-5,8-dihydroxyanthrachinon der Formel (II) 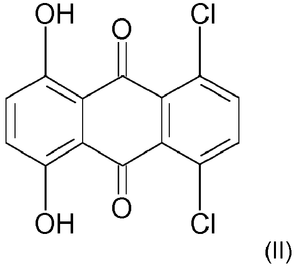 mit einer Verbindungen der Formel (IIIa) und/oder (IIIb) 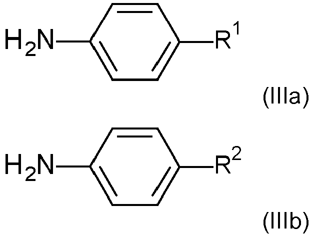 worin
R¹ und R² die für Formel (I) angegebene Bedeutung haben,
und mindestens einer Base, dadurch gekennzeichnet, dass die Umsetzung in Gegenwart von mindestens einem 5- oder 6-gliedrigen cyclischen Harnstoff-Derivat der Formel (IV) wie weiter unten definiert durchgeführt wird.

Unter dem Begriff Alkyl ist beispielsweise geradkettiges oder verzweigtes C₁-C₆-Alkyl, vorzugsweise geradkettiges oder verzweigtes C₁-C₄-Alkyl, insbesondere Methyl, Ethyl, nund iso-Propyl, sowie n-, iso- und tert.-Butyl zu verstehen. Die genannten Alkylreste können unsubstituiert, oder ein- oder mehrfach, gleich oder verschieden substituiert sein. Als Substituenten für die genannten Alkylreste kommen beispielsweise Halogen, wie Chlor, Brom oder Fluor, sowie Hydroxy, Cyano, Amino und C₁-C₆-Alkoxy in Frage.

Unter dem Begriff Alkoxy ist beispielsweise geradkettiges oder verzweigtes C₁-C₆-Alkoxy, vorzugsweise geradkettiges oder verzweigtes C₁-C₄-Alkoxy, insbesondere Methoxy, Ethoxy, n- und iso-Propoxy, sowie n- iso- und tert.-Butoxy zu verstehen. Die genannten Alkoxyreste können unsubstituiert, oder ein- oder mehrfach, gleich oder verschieden substituiert sein. Als Substituenten für die genannten Alkoxyreste kommen beispielsweise Halogen, wie Chlor, Brom oder Fluor, sowie Hydroxy, Cyano, Amino und C₁-C₆-Alkoxy in Frage.

Vorzugsweise dient das erfindungsgemäße Verfahren zur Herstellung von Verbindungen der Formel (I), worin R¹ und R² gleich sind und für C₁-C₄-Alkyl, insbesondere für Methyl, Ethyl, n- und iso-Propyl, sowie n- iso- und tert.-Butyl stehen.

Insbesondere dient das erfindungsgemäße Verfahren zur Herstellung von Verbindungen der Formel (I), worin R¹ und R² gleich sind und für C₁-C₄-Alkyl, insbesondere für Methyl, Ethyl, n- und iso-Propyl, sowie n- iso- und tert.-Butyl stehen.

Ganz besonders bevorzugt dient das erfindungsgemäße Verfahren zur Herstellung von Verbindungen der Formel (I), worin R¹ und R² gleich sind und für n-, iso- und tert.-Butyl stehen.

In einer alternativen Ausführungsform dient das erfindungsgemäße Verfahren zur Herstellung von Verbindungen der Formel (I), worin R¹ und R² gleich sind und die für Formel (I) angegebenen allgemeinen und bevorzugten Bedeutungen haben.

Das 1,4-Dichlor-5,8-dihydroxyanthrachinon der Formel (II) wird im Allgemeinen in reiner Form für das erfindungsgemäße Verfahren eingesetzt. Allerdings ist es auch möglich, mit dem gleich guten Ergebnis diese Verbindung als technische Ware einzusetzen. Diese technische Ware enthält dann neben dem Hauptbestandteil 1,4-Dichlor-5,8-dihydroxyanthrachinon im Allgemeinen noch 15 bis 30 % an Verunreinigungen in Form von höher substituierten Produkten und/oder zur Hauptkomponente isomere Produkte.

Bevorzugt wird für das erfindungsgemäße Verfahren 1,4-Dichlor-5,8-dihydroxyanthrachinon in Form dieser technischen Ware eingesetzt. Bei der erfindungsgemäßen Umsetzung mit den Aminen der Formel (III) werden auch die Chloratome der, in der technischen Ware enthaltenen, Nebenprodukte durch Aminogruppen ersetzt. Diese Reaktionsprodukte verbleiben allerdings nach der erfindungsgemäßen Umsetzung in der Mutterlauge und können mit dieser von den erfindungsgemäß hergestellten Verbindungen der Formel (I) abgetrennt werden und stellen somit keine Beeinträchtigung für die Qualität des Endproduktes dar.

1,4-Dichlor-5,8-dihydroxyanthrachinon kann in einer Ausbeute von etwa 80 % d. Th. durch Chlorierung von 1,4-Dihydroxyanthrachinon in Gegenwart von Borsäure und Jod in 30%igem Oleum hergestellt werden (vgl. O. Bayer, Methoden der Organischen Chemie (Houben-Weyl), 4. Auflage 1979, Band VII / 3c, Seite 121 f). Der Reinheitsgehalt einer solchermaßen hergestellten technischen Ware beträgt üblicherweise etwa 80 %.

Bevorzugt wird zur Durchführung des erfindungsgemäßen Verfahrens mindestens eine Verbindung der Formeln (IIIa) und/oder (IIIb) eingesetzt, worin R¹ und R² für C₁-C₄-Alkyl stehen.

Geeignete Verbindungen der Formeln (IIIa) und (IIIb) sind beispielsweise 4-Methylanilin, 4-Ethylanilin, 4-n-Propylanilin, 4-Isopropylanilin, 4-n-Butylanilin, 4-Isobutylanilin und 4-tert.-Butylanilin. Bevorzugt wird als Verbindung der Formeln (IIIa) und (IIIb) 4-n-Butylanilin, 4-Isobutylanilin und/oder 4-tert.Butylanilin eingesetzt, insbesondere wird 4-tert.Butylanilin eingesetzt.

Zur Durchführung des erfindungsgemäßen Verfahrens wird im allgemeinen pro Mol an 1,4-Dichlor-5,8-dihydroxyanthrachinon der Formel (II) ein Mol an mindestens einer Verbindung der Formeln (IIIa) und/oder (IIIb) eingesetzt, vorzugsweise zuzüglich eines Überschusses von 10 bis 50 Gew.-%, besonders bevorzugt von 10 bis 30 Gew.-%, insbesondere von 10 bis 20 Gew.-%, jeweils bezogen auf 1 Mol der Gesamtmenge an Verbindungen (IIIa) und (IIIb).

Die Amine der Formel (IIIa) und (IIIb) sind bekannte Handelsprodukte und können beispielsweise von der Fa. Lanxess Deutschland GmbH bezogen werden.

Das erfindungsgemäße Verfahren wird in Gegenwart von mindestens einem 5- oder 6-gliedrigen cyclischen Harnstoff-Derivat der Formel (IV) durchgeführt 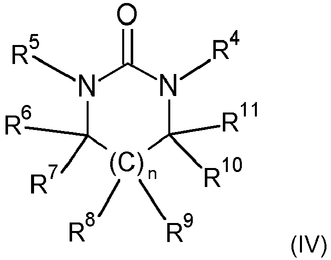 worin
R⁴ und R⁵ unabhängig voneinander jeweils für unsubstituiertes C₁-C₆-Alkyl stehen,
R⁶ bis R¹¹ unabhängig voneinander jeweils für Wasserstoff, C₁-C₆-Alkyl oder Phenyl stehen und
n für Null oder Eins steht.
Im Falle von n = Null bilden die freien Kohlenstoff-Valenzen eine Kohlenstoff-Kohlenstoff-Einfachbindung unter Bildung eines Fünfringes aus.

Besonders bevorzugt ist die Verwendung von einem oder mehreren cyclischen Harnstoff-Derivaten der Formel (IV) worin
R⁴ und R⁵ unabhängig voneinander jeweils für unsubstituiertes C₁-C₄-Alkyl stehen,
R⁶ bis R¹¹ unabhängig voneinander jeweils für Wasserstoff oder C₁-C₄-Alkyl stehen,
   und
n für Null oder Eins steht.

Ebenfalls besonders bevorzugt ist die Verwendung von einem oder mehreren cyclischen Harnstoff-Derivaten der Formel (IV) worin
R⁴ und R⁵ unabhängig voneinander jeweils für unsubstituiertes C₁-C₄-Alkyl stehen,
R⁶ bis R¹¹ unabhängig voneinander jeweils für Wasserstoff oder unsubstituiertes C₁-C₄-Alkyl stehen,
   und
n für Null oder Eins steht.

Ganz besonders bevorzugt ist die Verwendung von einem oder mehreren cyclischen Harnstoff-Derivaten der Formel (IV) worin
R⁴ und R⁵ gleich sind und für Methyl, Ethyl, n-Propyl, iso-Propyl, n-Butyl, iso-Butyl, tert.-Butyl, Hydroxymethyl, Hydroxyethyl, Methoxy oder Ethoxy stehen,
R⁶ bis R¹¹ unabhängig voneinander jeweils für Wasserstoff oder Methyl stehen,
   und
n für Null oder Eins steht.

Ebenfalls ganz besonders bevorzugt ist die Verwendung von einem oder mehreren cyclischen Harnstoff-Derivaten der Formel (IV) worin
R⁴ und R⁵ gleich sind und für Methyl, Ethyl, n-Propyl, iso-Propyl, n-Butyl, iso-Butyl oder tert.-Butyl, stehen,
R⁶ bis R¹¹ unabhängig voneinander jeweils für Wasserstoff oder Methyl stehen,
   und
n für Null oder Eins steht.

Insbesondere bevorzugt ist die Verwendung von cyclischen Harnstoff-Derivaten aus der Reihe

N,N'-Dimethylpropylenharnstoff (CAS-Nr. 7226-23-5), N,N'-Dimethylethylenharnstoff (CAS-Nr. 80-73-9), N,N'-Diethylpropylenharnstoff (CAS-Nr. 30826-85-8), N,N'-Dimethyl-5,5-dimethylpropylenharnstoff (CAS-Nr. 30879-82-4), N,N'-Di(n-propyl)propylenharnstoff (CAS-Nr. 30826-87-0), N,N'-Bis(hydroxymethyl)propylenharnstoff (CAS-Nr. 3270-74-4), N,N'-Bis(hydroxymethyl)ethylenharnstoff (CAS-Nr. 136-84-5, N,N'Bis(methoxymethyl)-ethylenharnstoff (CAS-Nr. 2669-72-9) und N,N'-Di(n-butyl)ethylenharnstoff (CAS-Nr. 4761-09-5).

Es ist möglich das erfindungsgemäße Verfahren in Gegenwart von einem oder mehreren der genannten cyclischen Harnstoff-Derivate durchzuführen. Dabei können die genannten cyclischen Harnstoff-Derivate einzeln oder in beliebiger Mischung untereinander eingesetzt werden. Bevorzugt ist es das erfindungsgemäße Verfahren in Gegenwart von nur einem der genannten Harnstoff-Derivate durchzuführen. Insbesondere bevorzugt ist die Verwendung von N,N'-Dimethylpropylenharnstoff, N,N'-Diethylpropylenharnstoff, N,N'-Dimethylethylenharnstoff und N,N'-Di(n-butyl)ethylenharnstoff.

Die genannten cyclischen Harnstoff-Derivate sind bekannt und können beispielsweise von der Firma BASF bezogen werden.

Im Allgemeinen wird zur Durchführung des erfindungsgemäßen Verfahrens pro Mol an 1,4-Dichlor-5,8-dihydroxyanthrachinon der Formel (II) 10 bis 30 Mol, vorzugsweise 12 bis 20 Mol mindestens eines cyclischen Harnstoff-Derivats eingesetzt.

Die zur Durchführung des erfindungsgemäßen Verfahrens eingesetzten cyclischen Harnstoff-Derivate dienen als Lösungsmittel für die Umsetzung von 1,4-Dichlor-5,8-dihydroxyanthrachinon mit den Aminen der Formel (III). Es ist bevorzugt das erfindungsgemäße Verfahren ausschließlich in Gegenwart dieser Lösungsmittel durchzuführen. Es ist allerdings möglich zur Durchführung des erfindungsgemäßen Verfahrens weitere Lösungsmittel wie z.B. N-Methylpyrrolidon, Chlorbenzol, 1,2-Dichlorbenzol, 1,2,4-Trichlorbenzol, Xylol, Nitrobenzol und Phenol oder Mischungen davon mit zu verwenden.

Das erfindungsgemäße Verfahren wird in Gegenwart mindestens einer Base durchgeführt. Bevorzugte Basen sind Trikaliumphosphat, Trinatriumphosphat, Dikaliumhydrogenphosphat, Dinatriumhydrogenphosphat, Kaliumdihydrogenphosphat, Natriumdihydrogenphosphat sowie Amine wie Triethanolamin und Diethanolamin oder Mischungen davon.
Besonders bevorzugt sind Dikaliumhydrogenphosphat und Dinatriumhydrogenphosphat.

Zur Durchführung des erfindungsgemäßen Verfahrens werden im Allgemeinen 0,5 bis 2,5 Äquivalente an Base, bevorzugt 0,5 bis 1 Äquivalent pro in den Verbindungen der Formel (II) auszutauschenden Chlor-Atomen bzw. pro Mol freiwerdende Salzsäure eingesetzt.

Das erfindungsgemäße Verfahren wird im Allgemeinen in einem Temperaturbereich von 100 bis 200 °C, bevorzugt von 120 bis 180 °C durchgeführt.

Das erfindungsgemäße Verfahren wird im Allgemeinen bei Umgebungsdruck durchgeführt. Es ist jedoch auch möglich das Verfahren in einem Druckbereich von 500 bis 2000 hPa, bevorzugt von 800 bis 1200 hPa durchzuführen. Unter Umgebungsdruck ist ein Luftdruck im Bereich von etwa 925 hPa bis 1070 hPa zu verstehen.

Im Allgemeinen wird das erfindungsgemäße Verfahren so durchgeführt, dass mindestens eines der als Lösungsmittel fungierenden cyclischen Harnstoffderivate vorgelegt wird, dann das 1,4-Dichlor-5,8-dihydroxyanthrachinon und mindestens eine Verbindung (IIIa) und/oder (IIIb) zugegeben werden und anschließend die Base zugegeben wird. Nach beendeter Umsetzung wird die Reaktionsmischung in üblicher Weise aufgearbeitet. Die Aufarbeitung erfolgt in bekannter Weise indem das gebildete 1,4-Diphenylamino-5,8-dihydroxyanthrachinon der Formel (I) aus dem Reaktionsmedium ausgefällt wird. Die Ausfällung kann durch Zugabe mindestens eines polaren Lösungsmittels, beispielsweise solche aus der Reihe der Alkohole, vorzugsweise Methanol, vervollständigt werden. Anschließend kann der Farbstoff der Formel (I) in üblicher Weise isoliert werden z.B. durch Filtration über eine Saugnutsche oder eine Filterpresse. Zur weiteren Reinigung kann der isolierte Farbstoff mit Wasser und/oder polaren Lösungsmitteln, vorzugsweise mit Wasser und Methanol, gewaschen werden. Danach wird der Farbstoff üblicherweise getrocknet, vorzugsweise bei einer Temperatur im Bereich von 80 bis 100 °C und bei einem Druck im Bereich von 100 bis 130 hPa.

Die Verunreinigungen an unerwünschten Nebenprodukten bleiben bei der erfindungsgemäßen Durchführung des Verfahrens in der Mutterlauge gelöst und werden bei der Isolierung mittels Filtration und Waschen des Rückstandes durch Verdrängung der Mutterlauge nahezu vollständig entfernt.

Das erfindungsgemäße Verfahren liefert die Verbindungen der Formel (I) in sehr guter Ausbeute von 95 bis 100 %, vorzugsweise >95 %, dabei beträgt der Gehalt an Verunreinigungen durch Amine der Formeln (IIIa) und/oder (IIIb) weniger als 600 ppm, vorzugsweise weniger als 300 ppm.

Bevorzugt dient das erfindungsgemäße Verfahren zur Herstellung von Verbindungen der Formel (I) worin R¹ und R² gleich sind und jeweils für n-Butyl, iso-Butyl oder tert.-Butyl stehen, insbesondere zur Herstellung von 1,4-Bis(4-tert.-butyl-phenylamino)-5,8-dihydroxyanthrachinon (Solvent Green 28, CAS-Nr. 4851-50-7, EINECS-Nr. 225-443-9). Die Verbindungen der Formel (I) stellen wertvolle Farbstoffe zum transparenten und gedeckten Färben von Kunststoffen wie Polystyrol, Polyamid, Polycarbonat (PC), Polyethylen, Polyester, z.B. Polyethylenterephtalat (PET) oder Polyethylenbutylat sowie Polymethylmethacrylat (PMMA), Acrylnitril-Butadien-Styrol-Copolymere (ABS) und ABS-PC-Blends dar. Sie können sowohl zur Massefärbung als auch zur Spinnfärbung der genannten Kunststoffe eingesetzt werden.

Die Verbindungen der Formel (I), insbesondere des so hergestellten 1,4-Bis(4-tert.-butyl-phenylamino)-5,8-dihydroxyanthrachinon dienen zum Färben von Kunststoffen in der Masse.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei dem der Farbstoff bereits Ausgangskomponenten zur Herstellung des Kunststoffes, z.B. Monomeren vor der Polymerisation, zugesetzt wird.

Besonders bevorzugte Kunststoffe sind Polystyrol, Polyamid, Polycarbonat (PC), Polyethylen, Polyester, z.B. Polyethylenterephtalat (PET) oder Polyethylenbutylat sowie Polymethylmethacrylat (PMMA), Acrylnitril-Butadien-Styrol-Copolymere (ABS) und ABS-PC-Blends.

Überraschenderweise zeichnen sich die mit dem erfindungsgemäßen Verfahren hergestellten Verbindungen der Formel (I), insbesondere das erfindungsgemäß hergestellte 1,4-Bis(4-tert.-butyl-phenylamino)-5,8-dihydroxyanthrachinon, durch eine verbesserte Farbbrillanz und eine verbesserte Farbstärke aus.

Der Farbstoff 1,4-Bis(4-tert.-butylphenylamino)-5,8-dihydroxyanthrachinon (Solvent Green 28), kann mit dem erfindungsgemäßen Verfahren mit einer Brillanz von 0,3 bis 0,8 dC, vorzugsweise von 0,4 bis 0,7 dC, gemessen nach DIN EN ISO 11664-4, und/oder einer Farbstärke von 100 bis 110 %, vorzugsweise von 105 bis 109 %, gemessen nach DIN 55986 und DIN EN ISO 11664-4 hergestellt werden.

Die folgenden Beispiele sollen die vorliegende Erfindung erläutern ohne sie jedoch darauf zu beschränken. Alle Prozentangaben beziehen sich auf das Gewicht, sofern nichts anderes angegeben ist.

### Beispiele:

### Beispiel 1

### Herstellung von 1,4-Bis(4-tert.-butyl-phenylamino)-5,8-dihydroxyanthrachinon (analog EP-A 1 074 586, nicht erfindungsgemäß)

In einem Glasreaktor wurden unter Stickstoff-Überleitung 100 g (1010 mMol) N-Methylpyrrolidon vorgelegt und auf 70 °C aufgeheizt. Danach wurden 20 g (134 mMol) p-tert.-Butylanilin zugegeben. Anschließend wurden 17 g (44 mMol) 5,8-Dichlor-1,4-dihydroxyanthrachinon (technische Ware, 80 %ig) und 15,7 g (110,5 mMol) Dinatriumhydrogenphosphat zugesetzt und das Reaktionsgemisch auf 150 °C aufgeheizt. Bei dieser Temperatur wurde die Reaktionsmischung für 3 Stunden gerührt und danach auf 180 °C aufgeheizt undbei dieser Temperatur weitere 10 Stunden gerührt. Nach beendeter Umsetzung wurde das Reaktionsgemisch auf 100 °C abgekühlt und 30 g Methanol zugegeben. Dieses Gemisch wurde bei 80 °C eine Stunde gerührt. Nach Abkühlung auf 50 °C wurde über eine Nutsche filtriert. Der Filterkuchen wurde zunächst mit 450 g warmem Methanol und danach mit 1500 ml warmem Wasser gewaschen. Das isolierte und gewaschene Produkt wurde dann in einem Vakuumtrockenschrank bei 70 °C und 150 hPa getrocknet.
Ausbeute 22 g (93,5 % d. Th.)

### Beispiel 2

### Herstellung von 1,4-Bis(4-tert.-butyl-phenylamino)-5,8-dihydroxyanthrachinon (analog EP-A 1 074 586, nicht erfindungsgemäß)

Die Umsetzung und Aufarbeitung erfolgte genau wie in Beispiel 1, jedoch wurden die 100 g N-Methylpyrrolidon ersetzt durch 100 g (680 mMol) 1,2-Dichlorbenzol.
Ausbeute 23 g (98 % d. Th.)

### Beispiel 3

### Herstellung von 1,4-Bis(4-tert.-butyl-phenylamino)-5,8-dihydroxyanthrachinon (analog EP-A 1 074 586, nicht erfindungsgemäß)

Die Umsetzung und Aufarbeitung erfolgte genau wie in Beispiel 1 angegeben, jedoch wurden die 100 g N-Methylpyrrolidon ersetzt durch 100 g (551 mMol) 1,2,4-Trichlorbenzol.
Ausbeute 22,6 g (96,1 % d. Th.)

### Beispiel 4

### Herstellung von 1,4-Bis(4-tert.-butyl-phenylamino)-5,8-dihydroxyanthrachinon (analog EP-A 1 074 586, nicht erfindungsgemäß)

Die Umsetzung und Aufarbeitung erfolgte genau wie in Beispiel 1 angegeben, jedoch wurden die 100 g N-Methylpyrrolidon ersetzt durch 100 g (813 mMol) Nitrobenzol.
Ausbeute 22,2 g (94,7 % d. Th.)

### Beispiel 5

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (781 mMol) N,N'-Dimethylpropylenharnstoff.
Ausbeute 23,1 g (98,5 % d. Th.)

### Beispiel 6

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (877 mMol) N,N'-Dimethylethylenharnstoff.
Ausbeute 23 g (98 % d. Th.)

### Beispiel 7

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (515 mMol) N,N'-Di(n-butyl)ethylenharnstoff.
Ausbeute 22,8 g (97 % d. Th.)

### Beispiel 8

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (658 mMol) N,N'-Diethylpropylenharnstoff.
Ausbeute 22,9 g (97,5 % d. Th.)

### Beispiel 9

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (781 mMol) N,N'-Dimethylpropylenharnstoff und die 15,7 g Dinatriumhydrogenphosphat wurden ersetzt durch 19,2 g (110 mMol) Dikaliumhydrogenphosphat
Ausbeute 23,1 g (98,5 % d. Th.)

### Beispiel 10

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (877 mMol) N,N'-Dimethylethylenharnstoff und die 15,7 g Dinatriumhydrogenphosphat wurden ersetzt durch 19,2 g (110 mMol) Dikaliumhydrogenphosphat
Ausbeute 22,9 g (97,5 % d. Th.)

### Beispiel 11

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (781 mMol) N,N'-Dimethylpropylenharnstoff und die 15,7 g Dinatriumhydrogenphosphat wurden ersetzt durch 11,6 g (110 mMol) Diethanolamin.
Ausbeute 23 g (98 % d. Th.)

### Beispiel 12

### Herstellung von 1,4-Bis(4-tert.-butyl-phenylamino)-5,8-dihydroxyanthrachinon (erfindungsgemäß)

Die Umsetzung und Aufarbeitung erfolgte genau wie in Beispiel 1 angegeben, jedoch wurden die 100 g N-Methylpyrrolidon ersetzt durch 100 g (877 mMol) N,N'-Dimethylethylenharnstoff und die 15,7 g Dinatriumhydrogenphosphat wurden ersetzt durch 11,6 g (110 mMol) Diethanolamin.
Ausbeute 22,9 g (97,5 % d. Th.)

### Bestimmung von Farbstärke und Brillanz:

Zur Bestimmung der Farbstärke und Brillanz der erfindungsgemäßen und nicht erfindungsgemäßen Proben der Beispiele 1 bis 26 wurde jede Probe einer koloristischen Messung, jeweils gegenüber dem Standard Farbstoff Macrolex® Grün G (CAS.-Nr. 4851-50-7) (Handelsprodukt der Firma Lanxess Deutschland GmbH), gemäß der unten angegebenen Vorschrift unterzogen.

Mit 2 Gewichtsprozent Titandioxid eingefärbtes Polystyrol Granulat wurde im Umlufttrockner bei 120°C für 4 Stunden getrocknet. 0,5 kg des Granulats wurde mit 0,10 Gewichtsprozent Farbstoffpulver vermischt. Die Mischung wurde bei 280°C Massetemperatur mit einem Doppelwellen-Extruder extrudiert und anschließend wieder granuliert. Das eingefärbte Granulat wurde bei 120°C für 4 Stunden getrocknet. Aus dem getrocknetem Granulat wurden Musterplättchen von 4 cm x 6 cm x 0,2 cm auf einer Spritzgießmaschine bei 240°C Massetemperatur, 10 bar Staudruck und 60°C Formtemperatur hergestellt.

Nach frühestens zehn Spritzzyklen wurden Musterplättchen für die Farbmessung entnommen und mindestens 1 Stunde bei Raumtemperatur ruhen gelassen.

Mit einem d/8° Spektralphotometer wurden von den Musterplättchen Remissionsmessungen durchgeführt. Die Bestimmung der Farbstärke und der Restfarbabstände erfolgte nach DIN 55986, die Bestimmung der Brillanz nach DIN EN ISO 11664-4.

Die Ergebnisse der koloristischen Prüfungen für die nicht erfindungsgemäßen und die erfindungsgemäßen Beispiele sind in Tabelle 1 aufgeführt.

**Tabelle 1**

| Beispiel | Erfindunggemäß | Farbstärke[%] | Brillanz [dC] |
|---|---|---|---|
| 1 | nein | 100 | 0 |
| 2 | nein | 94 | -0,4 |
| 3 | nein | 95 | -0,3 |
| 4 | nein | 96 | -0,4 |
| 5 | ja | 109 | 0,8 |
| 6 | ja | 107 | 0,7 |
| 7 | ja | 105 | 0,5 |
| 8 | ja | 105 | 0,4 |
| 9 | ja | 107 | 0,6 |
| 10 | ja | 106 | 0,6 |
| 11 | ja | 107 | 0,7 |
| 12 | ja | 105 | 0,5 |

### Fazit

Die erfindungsgemäß hergestellten Verbindungen der Beispiele 5 bis 12 weisen durchgehend höhere Farbstärken und höhere Brillanz im Vergleich zu den nichterfindungsgemäß hergestellten Verbindungen der Beispiele 1 bis 4 auf.

Farbstärke und Brillanz sind wesentliche Farbstoff-Eigenschaften, die von Bedeutung sind im Hinblick auf seine anwendungstechnische Eignung. Je höher die Farbstärke einer Probe, desto weniger Farbstoff muss zur Färbung eingesetzt werden. Je höher die Farbbrillanz einer Probe, desto farbsatter wie die Färbung wahrgenommen.

## Patentansprüche

1. Verfahren zur Herstellung von
Verbindungen der Formel (I) 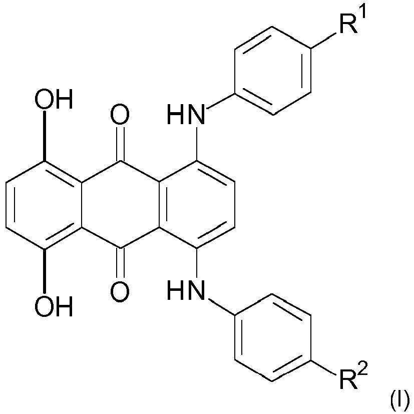 worin
R¹ und R² gleich sind und für C₁-C₆-Alkyl stehen,
durch Umsetzung von 1,4-Dichlor-5,8-dihydroxyanthrachinon der Formel (II) 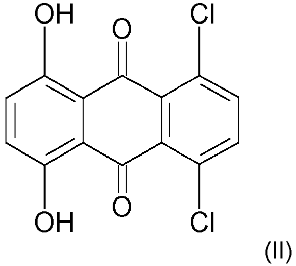 mit mindestens einer Verbindungen der Formeln (IIIa) und/oder (IIIb) 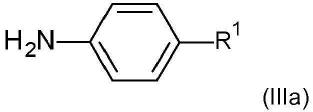 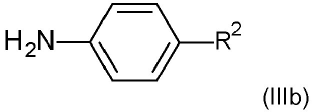 worin
R¹ und R² die für Formel (I) angegebene Bedeutung haben,
und mindestens einer Base, **dadurch gekennzeichnet, dass** die Umsetzung in Gegenwart von mindestens einem 5- oder 6-gliedrigen cyclischen Harnstoff-Derivat der Formel (IV) 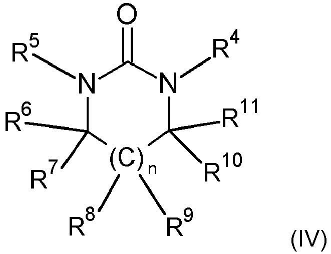 worin
R⁴ und R⁵ unabhängig voneinander jeweils für unsubstituiertes C₁-C₆-Alkyl stehen,
R⁶ bis R¹¹ unabhängig voneinander jeweils für Wasserstoff, C₁-C₆-Alkyl oder Phenyl stehen
und
n für Null oder Eins steht
durchgeführt wird.

2. Verfahren gemäß Anspruch 1, **dadurch gekennzeichnet, dass** in Formel (IV)
R⁴ und R⁵ unabhängig voneinander jeweils für unsubstituiertes C₁-C₄-Alkyl stehen,
R⁶ bis R¹¹ unabhängig voneinander jeweils für Wasserstoff oder C₁-C₄-Alkyl stehen, und
n für Null oder Eins steht.

3. Verfahren gemäß Anspruch 1 oder 2, **dadurch gekennzeichnet, dass** in Formel (IV)
R⁴ und R⁵ gleich sind und für Methyl, Ethyl, n-Propyl, iso-Propyl, n-Butyl, iso-Butyl oder tert.-Butyl, stehen,
R⁶ bis R¹¹ unabhängig voneinander jeweils für Wasserstoff oder Methyl stehen,
und
n für Null oder Eins steht.

4. Verfahren gemäß wenigstens einem der Ansprüche 1 bis 3, **dadurch gekennzeichnet, dass** pro Mol an 1,4-Dichlor-5,8-dihydroxyanthrachinon der Formel (II) 10 bis 30 Mol, vorzugsweise 12 bis 20 Mol mindestens eines 5- oder 6-gliedrigen cyclischen Harnstoff-Derivats der Formel (IV) eingesetzt wird.

5. Verfahren gemäß wenigstens einem der Ansprüche 1 bis 4, **dadurch gekennzeichnet, dass** als Verbindung der Formel (IIIa) und (IIIb) 4-n-Butylanilin, 4-Isobutylanilin oder 4-tert.-Butylanilin eingesetzt wird.

6. Verfahren gemäß wenigstens einem der Ansprüche 1 bis 5, **dadurch gekennzeichnet, dass** pro Mol an 1,4-Dichlor-5,8-dihydroxyanthrachinon der Formel (II) insgesamt ein Mol an mindestens einer Verbindung der Formeln (IIIa) und (IIIb) eingesetzt wird.

7. Verfahren gemäß wenigstens einem der Ansprüche 1 bis 6, **dadurch gekennzeichnet, dass** die mindestens eine Base ausgewählt ist aus der Reihe Trikaliumphosphat, Trinatriumphosphat, Dikaliumhydrogenphosphat, Dinatriumhydrogenphosphat, Kaliumdihydrogenphosphat, Natriumdihydrogenphosphat, Triethanolamin und Diethanolamin.

8. Verfahren gemäß wenigstens einem der Ansprüche 1 bis 7, **dadurch gekennzeichnet, dass** es bei einer Temperatur im Bereich von 100 bis 200 °C, bevorzugt von 120 bis 180 °C durchgeführt wird.

9. Verfahren gemäß wenigstens einem der Ansprüche 1 bis 8, **dadurch gekennzeichnet, dass** es bei einem Druck im Bereich von 500 bis 2000 hPa, bevorzugt von 800 bis 1200 hPa, besonders bevorzugt bei Umgebungsdruck durchgeführt wird.

## Claims

1. Method for preparing
compounds of formula (I) 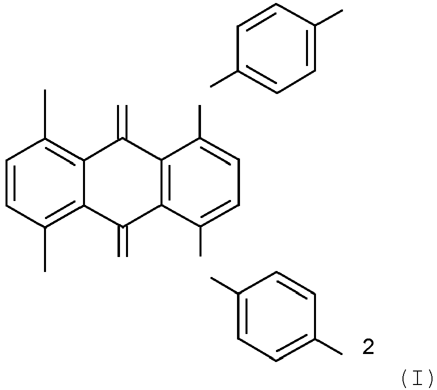 in which
R¹ and R² are the same and are C₁-C₆-alkyl,
by reacting 1,4-dichloro-5,8-dihydroxyanthraquinone of formula (II) 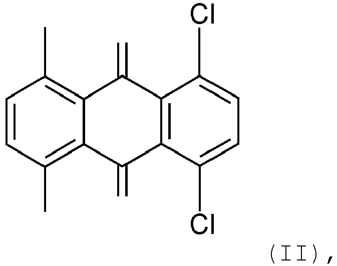 with at least one compound of formulae (IIIa) and/or (IIIb) 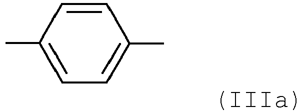 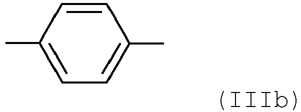 in which
R¹ and R² have the definition specified for formula (I),
and at least one base, **characterized in that** the reaction is carried out in the presence of at least one 5- or 6-membered cyclic urea derivative of formula (IV) 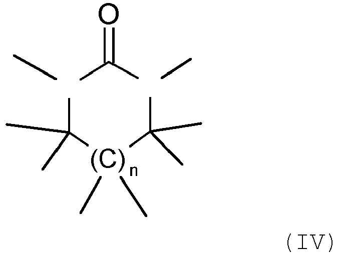 in which
R⁴ and R⁵ are each independently unsubstituted C₁-C₆-alkyl,
R⁶ to R¹¹ are each independently hydrogen, C₁-C₆-alkyl or phenyl,
and
n is zero or one.

2. Method according to Claim 1, **characterized in that**, in formula (IV)
R⁴ and R⁵ are each independently unsubstituted C₁-C₄-alkyl,
R⁶ to R¹¹ are each independently hydrogen or C₁-C₄-alkyl,
and
n is zero or one.

3. Method according to Claim 1 or 2, **characterized in that**, in formula (IV)
R⁴ and R⁵ are the same and are methyl, ethyl, n-propyl, isopropyl, n-butyl, isobutyl or tert-butyl,
R⁶ to R¹¹ are each independently hydrogen or methyl, and
n is zero or one.

4. Method according to at least one of Claims 1 to 3, **characterized in that**, per mole of 1,4-dichloro-5,8-dihydroxyanthraquinone of formula (II), 10 to 30 mol, preferably 12 to 20 mol of at least one 5- or 6-membered cyclic urea derivative of formula (IV) is used.

5. Method according to at least one of Claims 1 to 4, **characterized in that** 4-n-butylaniline, 4-isobutylaniline or 4-tert-butylaniline is used as compound of formula (IIIa) and (IIIb).

6. Method according to at least one of Claims 1 to 5, **characterized in that**, per mole of 1,4-dichloro-5,8-dihydroxyanthraquinone of formula (II), in total one mole of at least one compound of formulae (IIIa) and (IIIb) is used.

7. Method according to at least one of Claims 1 to 6, **characterized in that** the at least one base is selected from the series tripotassium phosphate, trisodium phosphate, dipotassium hydrogenphosphate, disodium hydrogenphosphate, potassium dihydrogenphosphate, sodium dihydrogenphosphate, triethanolamine and diethanolamine.

8. Method according to at least one of Claims 1 to 7, **characterized in that** it is conducted at a temperature in the range from 100 to 200°C, preferably from 120 to 180°C.

9. Method according to at least one of Claims 1 to 8, **characterized in that** it is conducted at a pressure in the range from 500 to 2000 hPa, preferably from 800 to 1200 hPa, particularly preferably at ambient pressure.

## Revendications

1. Procédé pour la préparation de composés de formule (I) 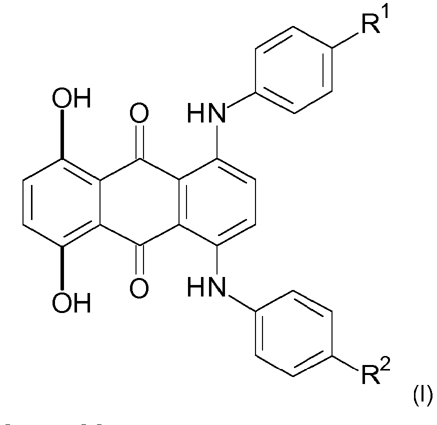 dans laquelle
R¹ et R² sont identiques et représentent C₁-C₆-alkyle, par transformation de 1,4-dichloro-5,8-dihydroxyanthraquinone de formule (II) 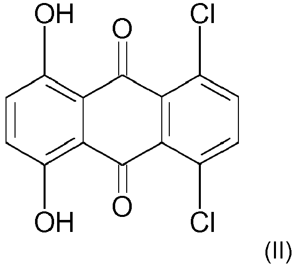 avec au moins un des composés des formules (IIIa) et/ou (IIIb) 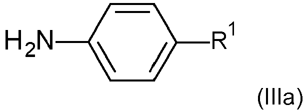 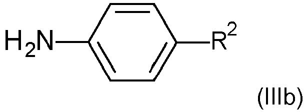 dans lesquelles
R¹ et R² ont la signification indiquée pour la formule (I),
et d'au moins une base, **caractérisé en ce que** la transformation est réalisée en présence d'au moins un dérivé cyclique à 5 ou 6 chaînons de l'urée de formule (IV) 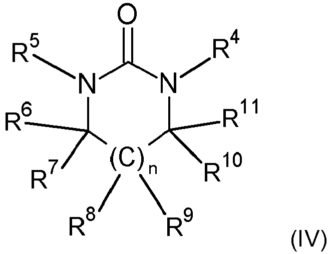 dans laquelle
R⁴ et R⁵ représentent, indépendamment l'un de l'autre, à chaque fois C₁-C₆-alkyle,
R⁶ à R¹¹ représentent, indépendamment les uns des autres, à chaque fois hydrogène, C₁-C₆-alkyle ou phényle et
n représente zéro ou un.

2. Procédé selon la revendication 1, **caractérisé en ce que**, dans la formule (IV),
R⁴ et R⁵ représentent, indépendamment l'un de l'autre, à chaque fois C₁-C₄-alkyle non substitué,
R⁶ à R¹¹ représentent, indépendamment les uns des autres, à chaque fois hydrogène ou C₁-C₄-alkyle et
n représente zéro ou un.

3. Procédé selon la revendication 1 ou 2, **caractérisé en ce que**, dans la formule (IV),
R⁴ et R⁵ sont identiques et représentent méthyle, éthyle, n-propyle, iso-propyle, n-butyle, iso-butyle ou tertbutyle,
R⁶ à R¹¹ représentent, indépendamment les uns des autres, à chaque fois hydrogène ou méthyle et
n représente zéro ou un.

4. Procédé selon au moins l'une quelconque des revendications 1 à 3, **caractérisé en ce qu'**on utilise, par mole de 1,4-dichloro-5,8-dihydroxyanthraquinone de formule (II), 10 à 30 moles, de préférence 12 à 20 moles d'au moins un dérivé cyclique à 5 ou 6 chaînons de l'urée de formule (IV).

5. Procédé selon au moins l'une quelconque des revendications 1 à 4, **caractérisé en ce qu'**on utilise, comme composé de formule (IIIa) et (IIIb), de la 4-n-butylaniline, de la 4-isobutylaniline ou de la 4-tert-butylaniline.

6. Procédé selon au moins l'une quelconque des revendications 1 à 5, **caractérisé en ce qu'**on utilise, par mole de 1,4-dichloro-5,8-dihydroxyanthraquinone de formule (II), au total une mole d'au moins un composé des formules (IIIa) et (IIIb).

7. Procédé selon au moins l'une quelconque des revendications 1 à 6, **caractérisé en ce que** ladite au moins une base est choisie dans la série phosphate tripotassique, phosphate trisodique, hydrogénophosphate dipotassique, hydrogénophosphate disodique, dihydrogénophosphate potassique, dihydrogénophosphate sodique, triéthanolamine et diéthanolamine.

8. Procédé selon au moins l'une quelconque des revendications 1 à 7, **caractérisé en ce qu'**il est réalisé à une température dans la plage de 100 à 200°C, de préférence de 120 à 180°C.

9. Procédé selon au moins l'une quelconque des revendications 1 à 8, **caractérisé en ce qu'**il est réalisé à une pression dans la plage de 500 à 2000 hPa, de préférence de 800 à 1200 hPa, de manière particulièrement préférée à la pression ambiante.
